# EUROPEAN PATENT APPLICATION

(11) **EP 2 361 935 A1**
(43) Date of publication of application: **31.08.2011**
(21) Application number: 10290080.0
(22) Date of filing: 18.02.2010
(51) Int. Cl.: C07K 16/28

(54) **Anti-CD28 humanized antibodies**

(71) Applicant: TcL Pharma, 44035 Nantes Cedex (FR); Institut National de la Santé et de la Recherche Médicale (INSERM), 75013 Paris (FR)
(72) Inventor: The designation of the inventor has not yet been filed
(74) Representative: Vialle-Presles, Marie José

(57) **Abstract**

The invention relates to humanized antibodies directed against the human lymphocyte receptor CD28. When used in a monovalent form these antibodies are capable of blocking of the CD28/B7 interaction, without activating CD28.

These antibodies can be used in particular as therapeutic agents for blocking T cell activation through the CD28 receptor.

SEQUENCE LISTING

<110>  TcL PHARMA
       INSTITUT NATIONAL DE LA SANTE ET DE LA RECHERCHE MEDICALE
       (INSERM)

<120>  ANTI-CD28 HUMANIZED ANTIBODIES

<130>  MJP/11 - F2173/3 EP

<160>  6

<170>  PatentIn version 3.3

<210>  1
<211>  120
<212>  PRT
<213>  Artificial

<220>
<223>  Synthetic construct

<400>  1

Val Gln Leu Gln Gln Ser Gly Ala Glu Leu Lys Lys Pro Gly Ala Ser
1               5                   10                  15

Val Lys Val Ser Cys Lys Ala Ser Gly Tyr Thr Phe Thr Glu Tyr Ile
            20                  25                  30

Ile His Trp Ile Lys Leu Arg Ser Gly Gln Gly Leu Glu Trp Ile Gly
        35                  40                  45

Trp Phe Tyr Pro Gly Ser Asn Asp Ile Gln Tyr Asn Ala Lys Phe Lys
    50                  55                  60

Gly Lys Ala Thr Leu Thr Ala Asp Lys Ser Ser Ser Thr Val Tyr Met
65                  70                  75                  80

Glu Leu Thr Gly Leu Thr Pro Glu Asp Ser Ala Val Tyr Phe Cys Ala
                85                  90                  95

Arg Arg Asp Asp Phe Ser Gly Tyr Asp Ala Leu Pro Tyr Trp Gly Gln
            100                 105                 110

Gly Thr Leu Val Thr Val Ser Ala
        115                 120

<210>  2
<211>  108
<212>  PRT
<213>  Artificial

<220>
<223>  Synthetic construct


<220>
<221>  MISC_FEATURE
<222>  (96)..(96)
<223>  Xaa = Cys or Ala

<400>  2

Asp Ile Gln Met Thr Gln Ser Pro Ser Ser Leu Ser Ala Ser Val Gly
1               5                   10                  15


Asp Arg Val Thr Ile Thr Cys Lys Thr Asn Glu Asn Ile Tyr Ser Asn
            20                  25                  30


Leu Ala Trp Tyr Gln Gln Lys Asp Gly Lys Ser Pro Gln Leu Leu Ile
        35                  40                  45


Tyr Ala Ala Thr His Leu Val Glu Gly Val Pro Ser Arg Phe Ser Gly
    50                  55                  60


Ser Gly Ser Gly Thr Gln Tyr Ser Leu Thr Ile Ser Ser Leu Gln Pro
65                  70                  75                  80


Glu Asp Phe Gly Asn Tyr Tyr Cys Gln His Phe Trp Gly Thr Pro Xaa
                85                  90                  95


Thr Phe Gly Gly Gly Thr Lys Leu Glu Ile Lys Arg
            100                 105


<210>  3
<211>  750
<212>  DNA
<213>  Artificial

<220>
<223>  Synthetic construct


<220>
<221>  CDS
<222>  (1)..(750)

<400>  3
atg gaa tgg tgc tgg gtc ttt ctc ttc ctc ctg tca gta act gca ggt       48
Met Glu Trp Cys Trp Val Phe Leu Phe Leu Leu Ser Val Thr Ala Gly
1               5                   10                  15

gtc cac tcc aag gtc caa ctg cag cag tct gga gct gag ctg aag aaa       96
Val His Ser Lys Val Gln Leu Gln Gln Ser Gly Ala Glu Leu Lys Lys
            20                  25                  30

ccc ggg gcg tcg gtg aaa gtc tcc tgc aag gcg tct ggt tac acc ttc 144
Pro Gly Ala Ser Val Lys Val Ser Cys Lys Ala Ser Gly Tyr Thr Phe
        35                  40                  45

act gaa tat att ata cac tgg ata aag ctg agg tct gga cag ggt ctt 192
Thr Glu Tyr Ile Ile His Trp Ile Lys Leu Arg Ser Gly Gln Gly Leu
    50                  55                  60

gag tgg att ggg tgg ttt tac cct gga agt aat gat ata cag tac aat 240
Glu Trp Ile Gly Trp Phe Tyr Pro Gly Ser Asn Asp Ile Gln Tyr Asn
65                  70                  75                  80

gcg caa ttc aag ggc aag gcc aca ttg act gcg gac aaa tcc tcc agc 288
Ala Gln Phe Lys Gly Lys Ala Thr Leu Thr Ala Asp Lys Ser Ser Ser
                85                  90                  95

acc gtc tat atg gaa ctt act gga ttg aca ccc gag gac tct gcg gtc 336
Thr Val Tyr Met Glu Leu Thr Gly Leu Thr Pro Glu Asp Ser Ala Val
            100                 105                 110

tat ttt tgt gca aga cgc gac gat ttc tct ggt tac gac gcc ctt cct 384
Tyr Phe Cys Ala Arg Arg Asp Asp Phe Ser Gly Tyr Asp Ala Leu Pro
        115                 120                 125

tac tgg ggc caa ggg act ctg gtc act gtc tct gca gct agc acc aag 432
Tyr Trp Gly Gln Gly Thr Leu Val Thr Val Ser Ala Ala Ser Thr Lys
    130                 135                 140

ggc cca tcg gtc ttc ccc ctg gca ccc tcc tcc aag agc acc tct ggg 480
Gly Pro Ser Val Phe Pro Leu Ala Pro Ser Ser Lys Ser Thr Ser Gly
145                 150                 155                 160

ggc aca gcg gcc ctg ggc tgc ctg gtc aag gac tac ttc ccc gaa ccg 528
Gly Thr Ala Ala Leu Gly Cys Leu Val Lys Asp Tyr Phe Pro Glu Pro
                165                 170                 175

gtg acg gtg tcg tgg aac tca ggc gcc ctg acc agc ggc gtg cac acc 576
Val Thr Val Ser Trp Asn Ser Gly Ala Leu Thr Ser Gly Val His Thr
            180                 185                 190

ttc ccg gct gtc cta cag tcc tca gga ctc tac tcc ctc agc agc gtg 624
Phe Pro Ala Val Leu Gln Ser Ser Gly Leu Tyr Ser Leu Ser Ser Val
        195                 200                 205

gtg acc gtg ccc tcc agc agc ttg ggc acc cag acc tac atc tgc aac 672
Val Thr Val Pro Ser Ser Ser Leu Gly Thr Gln Thr Tyr Ile Cys Asn
    210                 215                 220

gtg aat cac aag ccc agc aac acc aag gtg gac aag aaa gtt gag ccc 720
Val Asn His Lys Pro Ser Asn Thr Lys Val Asp Lys Lys Val Glu Pro
225                 230                 235                 240

aaa tct tgt gac aaa act cac aca tgc taa 750
Lys Ser Cys Asp Lys Thr His Thr Cys
                245

<210> 4
<211> 249
<212> PRT
<213> Artificial

<220>
<223>  Synthetic Construct

<400>  4

Met Glu Trp Cys Trp Val Phe Leu Phe Leu Leu Ser Val Thr Ala Gly
1               5                   10                  15

Val His Ser Lys Val Gln Leu Gln Gln Ser Gly Ala Glu Leu Lys Lys
            20                  25                  30

Pro Gly Ala Ser Val Lys Val Ser Cys Lys Ala Ser Gly Tyr Thr Phe
        35                  40                  45

Thr Glu Tyr Ile Ile His Trp Ile Lys Leu Arg Ser Gly Gln Gly Leu
    50                  55                  60

Glu Trp Ile Gly Trp Phe Tyr Pro Gly Ser Asn Asp Ile Gln Tyr Asn
65                  70                  75                  80

Ala Gln Phe Lys Gly Lys Ala Thr Leu Thr Ala Asp Lys Ser Ser Ser
                85                  90                  95

Thr Val Tyr Met Glu Leu Thr Gly Leu Thr Pro Glu Asp Ser Ala Val
            100                 105                 110

Tyr Phe Cys Ala Arg Arg Asp Asp Phe Ser Gly Tyr Asp Ala Leu Pro
        115                 120                 125

Tyr Trp Gly Gln Gly Thr Leu Val Thr Val Ser Ala Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys
                245

<210>  5
<211>  705
<212>  DNA
<213>  Artificial

<220>
<223>  Synthetic construct

<220>
<221>  CDS
<222>  (1)..(705)

<400>  5
atg agt gtg ccc act cag gtc ctg ggg ttg ctg ctg ctg tgg ctt aca       48
Met Ser Val Pro Thr Gln Val Leu Gly Leu Leu Leu Leu Trp Leu Thr
1               5                   10                  15

gat gcc aga tgt gac atc cag atg act cag tct cca tct tcc cta tct       96
Asp Ala Arg Cys Asp Ile Gln Met Thr Gln Ser Pro Ser Ser Leu Ser
            20                  25                  30

gca tct gtg gga gac agg gtc acc atc acg tgt aaa aca aat gag aat      144
Ala Ser Val Gly Asp Arg Val Thr Ile Thr Cys Lys Thr Asn Glu Asn
        35                  40                  45

att tac agt aat tta gca tgg tat cag cag aaa gac gga aaa tct cct      192
Ile Tyr Ser Asn Leu Ala Trp Tyr Gln Gln Lys Asp Gly Lys Ser Pro
    50                  55                  60

cag ctc ctg atc tat gct gca aca cac tta gta gag ggt gtg cca tca      240
Gln Leu Leu Ile Tyr Ala Ala Thr His Leu Val Glu Gly Val Pro Ser
65                  70                  75                  80

agg ttc agt ggc agt gga tca ggc aca cag tat tcc ctc aca atc agc      288
Arg Phe Ser Gly Ser Gly Ser Gly Thr Gln Tyr Ser Leu Thr Ile Ser
                85                  90                  95

agc ctg cag cca gaa gat ttt ggg aat tat tac tgt caa cac ttt tgg      336
Ser Leu Gln Pro Glu Asp Phe Gly Asn Tyr Tyr Cys Gln His Phe Trp
            100                 105                 110

ggt act ccg tgc acg ttc gga ggg ggg acc aag ctg gaa ata aaa cgg      384
Gly Thr Pro Cys Thr Phe Gly Gly Gly Thr Lys Leu Glu Ile Lys Arg
        115                 120                 125

acg gtg gct gca cca tct gtc ttc atc ttc ccg cca tct gat gag cag      432
Thr Val Ala Ala Pro Ser Val Phe Ile Phe Pro Pro Ser Asp Glu Gln
    130                 135                 140

ttg aaa tct gga act gcc tct gtt gtg tgc ctg ctg aat aac ttc tat 480
Leu Lys Ser Gly Thr Ala Ser Val Val Cys Leu Leu Asn Asn Phe Tyr
145                 150                 155                 160

ccc aga gag gcc aaa gta cag tgg aag gtg gat aac gcc ctc caa tcg 528
Pro Arg Glu Ala Lys Val Gln Trp Lys Val Asp Asn Ala Leu Gln Ser
                165                 170                 175

ggt aac tcc cag gag agt gtc aca gag cag gac agc aag gac agc acc 576
Gly Asn Ser Gln Glu Ser Val Thr Glu Gln Asp Ser Lys Asp Ser Thr
            180                 185                 190

tac agc ctc agc agc acc ctg acg ctg agc aaa gca gac tac gag aaa 624
Tyr Ser Leu Ser Ser Thr Leu Thr Leu Ser Lys Ala Asp Tyr Glu Lys
        195                 200                 205

cac aaa gtc tac gcc tgc gaa gtc acc cat cag ggc ctg agt tcg ccc 672
His Lys Val Tyr Ala Cys Glu Val Thr His Gln Gly Leu Ser Ser Pro
    210                 215                 220

gtc aca aag agc ttc aac agg gga gag tgt taa 705
Val Thr Lys Ser Phe Asn Arg Gly Glu Cys
225                 230

<210> 6
<211> 234
<212> PRT
<213> Artificial

<220>
<223> Synthetic Construct

<400> 6

Met Ser Val Pro Thr Gln Val Leu Gly Leu Leu Leu Leu Trp Leu Thr
1               5                   10                  15

Asp Ala Arg Cys Asp Ile Gln Met Thr Gln Ser Pro Ser Ser Leu Ser
            20                  25                  30

Ala Ser Val Gly Asp Arg Val Thr Ile Thr Cys Lys Thr Asn Glu Asn
        35                  40                  45

Ile Tyr Ser Asn Leu Ala Trp Tyr Gln Gln Lys Asp Gly Lys Ser Pro
    50                  55                  60

Gln Leu Leu Ile Tyr Ala Ala Thr His Leu Val Glu Gly Val Pro Ser
65                  70                  75                  80

Arg Phe Ser Gly Ser Gly Ser Gly Thr Gln Tyr Ser Leu Thr Ile Ser
                85                  90                  95

Ser Leu Gln Pro Glu Asp Phe Gly Asn Tyr Tyr Cys Gln His Phe Trp
            100                 105                 110

Gly Thr Pro Cys Thr Phe Gly Gly Gly Thr Lys Leu Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

## Description

The present invention relates to humanized monoclonal antibodies binding CD28, to monovalent fragments thereof, and to their therapeutic uses, in particular in the context of regulating T cell activation.

Abnormal activation of T cells is involved in the pathogenesis of many autoimmune diseases, and also in transplant rejection phenomena, where they cause an immune response directed against the transplanted organ to develop.

One of the most important systems for regulating T lymphocyte activation is the molecular system B7/CD28/CTLA4. This system plays, for example, an essential role in the mechanisms of transplant rejection (WOODWARD et al., Transplantation, 66, 14-20, 1998). The molecules B7.1 (CD80) and B7.2 (CD86) borne by the APCs can activate the receptor CD28 and also the receptor CTLA4 of T lymphocytes. The activation of CD28 sends the T lymphocyte a positive signal which stimulates the cell; on the other hand, the activation of CTLA4 sends a negative signal which leads to a non-response (anergy) (FALLARINO et al., J. Exp. Med., 188, 205-210, 1998).

Resting T lymphocytes express a large amount of CD28 and very little CTLA4. When there is a first cognitive contact between an APC and a T lymphocyte, the CD28/B7 interaction is favored, which activates the cell. It is only several hours after the initiation of activation that, due to the increase in membrane expression of CTLA4, the affinity of which for B7 is 5 to 10 times greater than that of CD28, the B7/CD28 interaction shifts in favor of a B7/CTLA4 interaction.

Selective inhibition of the agonist signal given to the T cell by CD28, leaving the antagonist system consisting of the pair CTLA4/B7 intact, via specific blocking of the CD28/B7 interaction, would make it possible to prevent T lymphocyte activation. Such specific blocking of the CD28/B7 interaction can be obtained using some antibodies directed against CD28.

These antibodies are to be used in a monovalent form (for instance as Fab or scFv fragments), since when used in their divalent native form, their binding to CD28 brings about the dimerization and the activation of this receptor. Fab fragments result from the action of papain on an immunoglobulin molecule, and each contain a light chain and the first half of a heavy chain; scFv fragments consist of the variable portions of the heavy and light chains of a parent antibody, connected to one another via a flexible linker (CLACKSON et al., Nature, 352, 624-628, 1991), thus forming a single-chain protein.

One such antibody is antibody CD 28.3, produced by the hybridoma cell line CNCM I-I-2582, and disclosed in PCT application WO 02/051871. This antibody, when used in a monovalent form such as scFv fragments, is capable of blocking *in vitro* the CD28 receptor without activating it (PCT WO 02/051871; VANHOVE et al., Blood, 102, 564-70, 2003), and has shown also its efficiency *in vivo* in models of organ transplantation in mice and in primates (POIRIER et al., World Transplant Congress, Sydney, Australia. August 16-21, 2008 ; POIRIER et al, Sci Trans Med, 2:17, p17ra10, 2010).

A drawback of all monoclonal antibodies derived from murine sources, is their immunogenicity when administered to human subjects. They provoke anti-mouse immune response, which results in a lesser efficiency of the treatment, in particular when repeated administration is required.

This drawback can, in principle, be avoided by the use of humanized antibodies. The aim of humanization is to obtain a recombinant antibody which has similar antigen-binding properties as the mouse monoclonal antibody from which the complementarity-determining regions (CDRs) sequences were derived, and which is far less immunogenic in humans.

The CDRs are the portions of the variable domains of an antibody which directly contact the antigen and determine the antigen-binding specificity; the framework regions (FRs) which are located between the CDRs in the variable domains do not directly contact the antigen, but serves as a scaffold to maintain the global structure of the variable domains.

Several approaches to antibody humanization have been reported. The more widely used are based on "CDR grafting", which involves the transplantation of the CDRs of a murine antibody into appropriate human FRs. However, in many antibodies, some FR residues are important for antigen binding, because they influence the conformation of CDRs and thus their antigen binding properties, in particular the binding affinity. A loss in binding affinity is particularly detrimental in the case of an antibody intended to be used in a monovalent form which generally exhibit less affinity for the antigen than the native divalent antibody. Thus, in most cases it is further necessary, to obtain a sufficient binding affinity, to reintroduce one or several framework residues from the mouse antibody in the human FRs, with the risk of simultaneously bring back unwanted immunogenicity.

Another approach to antibody humanisation, called "de-immunization", involves the identification within the FRs regions of the antibody, of B-cell and T-cell epitopes recognized as "foreign" and therefore potentially immunogenic in humans, and to remove them by appropriate amino-acids substitutions. This approach however also entails the risk that FR residues important for antigen binding are deleted. Moreover, some immunogenic epitopes may lie in the CDRs and trying to remove them involves a very high risk of destroying not only the antigen-binding affinity but also the antigen-binding specificity of the antibody.

Therefore, a major issue in antibody humanisation is to determine which amino acid residues are critical for retaining the antigen-binding properties. Various methods have been proposed for predicting the more appropriate sites for substitution in the FRs regions. Although they provide general principles that may be of some help in the first steps of humanization, the final result greatly varies from an antibody to another. Thus, for a given antibody, it is very difficult to foretell which substitutions will provide the desired result. In the case wherein not only substitutions in the FRs, but also in the CDRs would be necessary to decrease satisfactorily the immunogenicity in humans, the final result becomes totally unpredictable.

The inventors have succeeded in producing humanized CD28.3 (hereinafter referred to as hCD28.3), with a low immunogenicity, and which, although it has several amino-acids substitutions including a non-conservative K→Q substitution in the CDR2 of the heavy chain, retains the CD28 binding properties of the parent mouse CD28.3. When used in a monovalent form, the hCD28.3 of the invention also retains the CD28 binding properties of the parent mouse CD28.3.

The present invention provides an anti-CD28 antibody, characterised in that:
- its heavy chain comprises a variable domain defined by the following polypeptide sequence: 
VQLQQSGAELKKPGASVKVSCKASGYTFTEYIIHWIKLRSGQGLEWIGWFYPGSNDI
QYNAKFKGKATLTADKSSSTVYMELTGLTPEDSAVYFCARRDDFSGYDALPYWGQG
TLVTVSA (SEQ ID NO: 1);
- its light chain comprises a variable domain defined by the following polypeptide sequence: 
DIQMTQSPSSLSASVGDRVTITCKTNENIYSNLAWYQQKDGKSPQLLIYAATHLVEGV
PSRFSGSGSGTQYSLTISSLQPEDFGNYYCQHFWGTPXTFGGGTKLEIKR (SEQ ID NO:
 2), wherein X = C or A.

The term "anti-CD28 antibody" herein refers to any antigen-binding protein having at least one antigen-binding site (consisting of the variable domains of the light chain and of the heavy chain) able to specifically bind human CD28. It encompasses antibodies in a divalent form (such as native immunoglobulin molecules or F(ab)'₂ fragments) with two CD28-binding sites, as well as antibodies in a monovalent form which have a single CD28-binding site, (for instance Fab, Fv and scFv fragments). In most cases, antibodies in a monovalent form will be preferred.

It also refers to recombinant antibodies comprising a CD28-binding site associated with one or more other polypeptide(s). By way of non-limitative examples, a hCD28.3 antibody of the invention may comprise, besides the variable domains defined above, one or more of the following components:
- a human constant region (Fc). This constant region can be selected among constant domains from any class of immunoglobulins, including IgM, IgG, IgD, IgA and IgE, and any isotype, including IgG 1, IgG2, IgG3 and IgG4. Preferred constant regions are selected among constant domains of IgG, in particular IgG4.
- a protein having pharmacological activity (for example a toxin);
- a protein which makes it possible to prolong the plasma half-life when it is administered *in vivo* under monovalent form as disclosed for instance in PCT WO 02/051871; advantageously said molecule can be the CH2-CH3 domains of an IgG molecule as disclosed in PCT/IB/2010/000196;
- one or more tag polypeptide(s).

The antibodies of the invention can also be conjugated with water soluble polymers such as polyethylene glycol (PEGylation). PEGylation is a classical way to enhance the pharmacokinetic properties of therapeutic polypeptides, and can be achieved by techniques known in the art.

The invention also encompasses a polynucleotide selected among:
a) a polynucleotide encoding a polypeptide of SEQ ID NO: 1;
b) a polynucleotide encoding a polypeptide of SEQ ID NO: 2;
c) a polypeptide encoding an hCD28.3 antibody of the invention, as defined above.

Polynucleotides of the invention generally also comprise additional sequences: for instance they may advantageously comprise a sequence encoding a leader sequence or signal peptide allowing secretion of said protein chain.

The present invention also encompasses recombinant vectors, in particular expression vectors, comprising a polynucleotide of the invention, associated with transcription- and translation-controlling elements which are active in the host cell chosen. Vectors which can be used to construct expression vectors in accordance with the invention are known in themselves, and will be chosen in particular as a function of the host cell intended to be used.

The present invention also encompasses host-cells transformed with a polynucleotide of the invention. Preferably, said host cell is transformed with a polynucleotide comprising a sequence encoding the heavy chain of a hCD28.3 antibody of the invention and a polynucleotide comprising a sequence encoding the light chain of a hCD28.3 antibody of the invention, and expresses said antibody. Said polynucleotides can be inserted in the same expression vector, or in two separate expression vectors.

Host cells which can be used in the context of the present invention can be prokaryotic or eukaryotic cells. Among the eukaryotic cells which can be used, mention will in particular be made of plant cells, cells from yeast, such as Saccharomyces, insect cells, such as Drosophila or Spodoptera cells, and mammalian cells such as HeLa, CHO, 3T3, C 127, BHK, COS, etc., cells.

The construction of expression vectors of the invention and the transformation of the host cells can be carried out by the conventional techniques of molecular biology.

Still another objet of the invention is a method for preparing a hCD28.3 antibody of the invention. Said method comprises culturing an host-cell transformed with a polynucleotide comprising a sequence encoding the heavy chain of a hCD28.3 antibody of the invention and a polynucleotide comprising a sequence encoding the light chain of a hCD28.3 antibody of the invention and recovering said antibody from said culture.

If the antibody is secreted by the host-cell, it can be recovered directly from the culture medium; if not, cell lysis will be carried out beforehand. The antibody can then be purified from the culture medium or from the cell lysate, by conventional procedures, known in themselves to those skilled in the art, for example by fractionated precipitation, in particular precipitation with ammonium sulfate, electrophoresis, gel filtration, affinity chromatography, etc.

The hCD28.3 antibodies of the invention can be used to obtain medicinal products. These medicinal products are also part of the object of the invention.

The present invention also comprises a therapeutic composition comprising a hCD28.3 antibody of the invention, together with a pharmaceutically acceptable carrier.

Preferably, said composition is a composition for parenteral administration, formulated to allow the administration of a dose of from 100 to 1000 mg, advantageously of from 350 to 700 mg of hCD28.3.

For instance, hCD28.3 antibodies of the invention can be used to obtain immunosuppressant medicinal products which selectively blocks T cell activation phenomena involving the CD28 receptor. Such immunosuppressant medicinal products which act by selective blocking of CD28 have applications in all T lymphocyte-dependent pathological conditions, including in particular transplant rejection, graft-versus-host disease, T lymphocyte-mediated autoimmune diseases, such as type I diabetes, rheumatoid arthritis or multiple sclerosis, and type IV hypersensitivity, which is involved in allergic phenomena and also in the pathogenesis of chronic inflammatory diseases, in particular following infection with a pathogenic agent (in particular leprosy, tuberculosis, leishmaniasis, listeriosis, etc.).

The present invention will be understood more clearly from the further description which follows, which refers to nonlimiting examples of the preparation and properties of a hCD28.3 antibody in accordance with the invention.

The construction of expression vectors of the invention and the transformation of host-cells can be made by the standard techniques of molecular biology.

A hCD28.3 antibody of the invention can be obtained by culturing a host cell containing an expression vector comprising a nucleic acid sequence encoding said antibody, under conditions suitable for the expression thereof, and recovering said antibody from the host cell culture.

The present invention will be further illustrated by the following additional description, which refers to examples illustrating the properties of hCD28.3 antibodies of the invention. It should be understood however that these examples are given only by way of illustration of the invention and do not constitute in any way a limitation thereof.

### EXAMPLE 1: CONSTRUCTION AND EUCARYOTIC EXPRESSION OF A hCD28.3 MONOVALENT ANTIBODY (Fab FRAGMENT)

### Heavy chain:

The sequence encoding the VH region of hCD28.3 (SEQ ID NO: 1) in fusion with the sequence encoding the human CH1 region (NCBI Accession number AAF03881) and with a sequence encoding the leader peptide of the heavy chain of the native murine CD28.3 antibody, was synthetized chemically, and introduced in the cloning vector pGA18 (Geneart) for amplification. The sequence was then excised by digestion with Kpnl/BamHI restriction enzymes and subcloned into the KpnI/BamHI sites of the plasmid pcDNA3.1-hygro (Invitrogen). Positive clones were amplified and purified by Midiprependotoxin free (Macherey-Nagel) for transfection step.

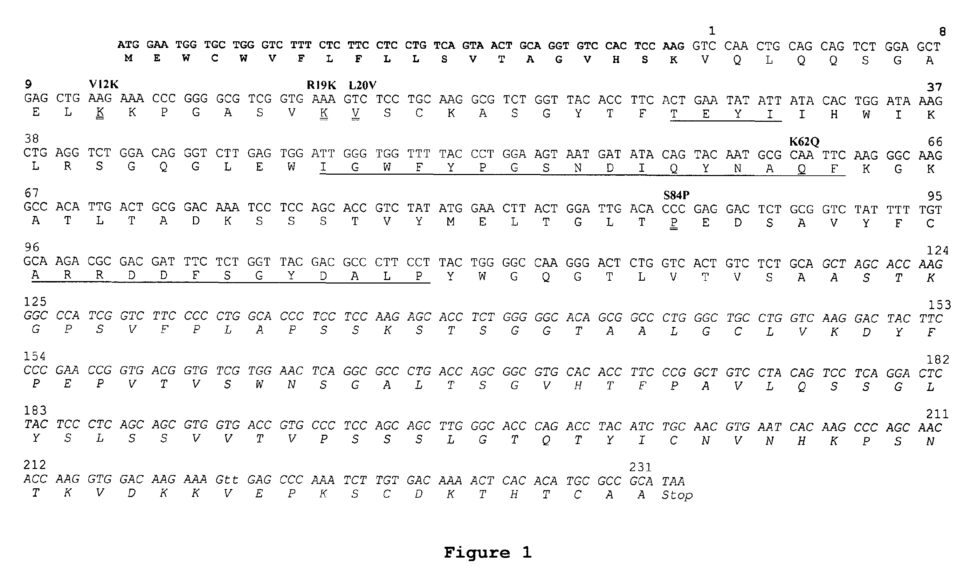

The resulting plasmid is designated pSignal-VH-hCH1. It comprises a construct containing the sequence encoding the VH region of hCD28.3 between the sequence encoding the CD28.3 heavy chain signal peptide and the sequence encoding the human CH1 region (NCBI Accession number AAF03881).The nucleotidic and amino acid sequences of this construct are shown on Figure 1. They are also represented as SEQ ID NO: 3 and SEQ ID NO: 4 in the enclosed sequence listing.

The signal peptide is represented in bold, and the human CH1 region is represented in italics. The CDRs of the parent CD28.3 antibody are underlined, and the substitutions made in the CD28.3 antibody VH region are underlined.

### Light chain:

The sequence encoding the VL region of hCD28.3 (SEQ ID NO: 2) in fusion with the sequence encoding the human c kappa region (NCBI accession number BAC01725) and with a sequence encoding the leader peptide of the light chain of the native murine CD28.3 antibody, was synthetized chemically, and introduced in the cloning vector pGA18 (Geneart) for amplification. The sequence was then excised by digestion with KpnI/BamHI restriction enzymes and subcloned into the Kpnl/BamHI sites of the plasmid pcDNA3.A-hygro (Invitrogen). Positive clones were amplified and purified by Midiprependotoxin free (Macherey-Nagel) for transfection step.

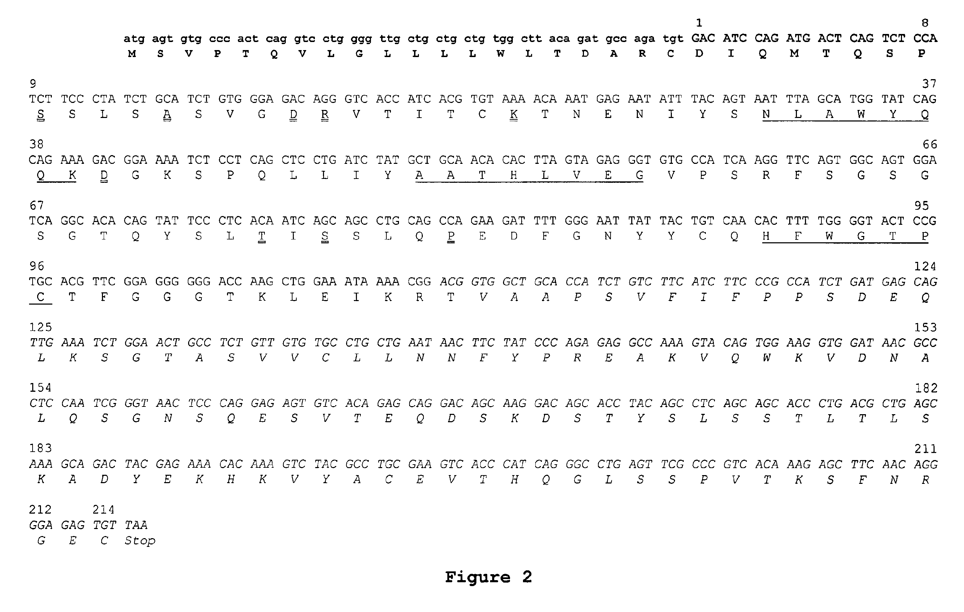

The resulting plasmid is designated pSignal-VL-hCK. It comprises a construct containing the sequence encoding the VL region of hCD28.3 between the sequence encoding the CD28.3 light chain signal peptide and the sequence encoding the human c kappa region (NCBI accession number BAC01725). The nucleotidic and amino acid sequences of this construct are shown on Figure 2. They are also represented as SEQ ID NO: 5 and SEQ ID NO: 6 in the enclosed sequence listing.

The signal peptide is represented in bold, and the human c kappa region is represented in italics. The CDRs of the parent CD28.3 antibody are underlined, and the substitutions made in the parent CD28.3 antibody VL region are underlined.

### Eucaryotic expression

COS cells were co-transfected with 2 µg (each) pSignal-VL-hCHI and pSignal-VH-hCH 1 using the Fugene lipofection kit (Roche Diagnostics, Basel, Switzerland) according to the manufacturer's instructions. Cultures were maintained for 3 days at 37°C, divided one third, and put back into culture for an additional 3 days, after which time the cell supernatants were collected.

The activity of the hCD28.3 monovalent antibody is evaluated directly in the supernatant by ELISA, as described in Example 2 below.

### EXAMPLE 2: DETECTION OF THE hCD28.3 Fab FRAGMENT BINDING ACTIVITY BY ELISA

The binding properties of the hCD28.3 Fab fragment have been compared with those obtained after transfection of Cos cells with plasmids coding for CD28.3 Fab (not humanized), using two ELISA assays

* First (Sandwich ELISA), the concentrations of the hCD28.3 and CD28.3 Fab fragments in the culture supernatants of transfected COS cells have been determined using a sandwich ELISA. Briefly, the anti-CD28 Fab contained in the supernatants are first captured by a rabbit polyclonal antibody, specific for the heavy and light variable domains of CD28.3 (obtained after immunization of rabbits with a single-chain-Fv containing the heavy and light variable domains of the native CD28.3, and purified by immunoadsorption on CD28.3 Fab-Sepharose) . The captured proteins are then revealed with a murine monoclonal antibody directed to the kappa chain of human IgG, followed by a polyclonal goat anti-mouse antibody labelled with peroxidase. Bound antibody was revealed by colorimetry using the TMB substrate, and read at 405 nm.

The OD corresponding to different dilutions of the supernatant are then compared to a standard curve obtained with known quantities of a CD28.3 Fab, called FR104, purified from culture supernatant of transformed CHO cells with standard techniques of chromatography, and dosed with a BCA (bisynchronic acid) assay. FR104 contains the native (not humanized), VH and VL regions of the CD28.3 antibody. Therefore, we can evaluate the amount of Fab proteins present in cell supernatants.

* Second (Binding ELISA), for testing the binding activity of hCD28.3 Fab fragments compared to CD28.3 Fab, chimeric human CD28/Fc (R&D Systems, Abingdon, United Kingdom) was used at 2 µg/ml in carbonate buffer 0.05M pH 9.2 to coat the wells (50 µL/well) of microtiter plates (Nunc Immunoplates) overnight at 4°C. These immobilized CD28 target molecules will bind only immunoreactive molecules with anti-CD28 activity.

The wells were then washed 3 times successively with 200 µL PBS-0.05% Tween, and saturated with 100µL PBS Tween 0.1 % BSA 1 % for 2 hours at 37°C.

Then, after 3 washings with 200 µL PBS-0.05%Tween, supernatants containing known concentrations of CD28.3 or hCD28.3 Fab fragments were added (50 µL/well) at different dilutions in PBS-0.1% Tween and incubated for 2 hours at 37°C. After 3 washings with 200 µL PBS-0.05%Tween, a murine monoclonal antibody directed to the kappa chain of human IgG, (1/10000 dilution) was added (1 hour, 37°C), followed by peroxidase-conjugated goat anti-mouse antibodies (1/2000 dilution), followed by colorimetric revelation using the TMB substrate and reading at 405 nm.

Then the results are plotted as the absorbance (Y axis), measured with the binding ELISA, according to the Fab concentration (X axis), measured with the sandwich ELISA. An AC50 (Antibody Concentration 50) is determined after calculating the slope of the curve in its linear range as the concentration of the anti-CD28 Fab needed to reach 50% of the maximal optical density (OD) in the binding assay.

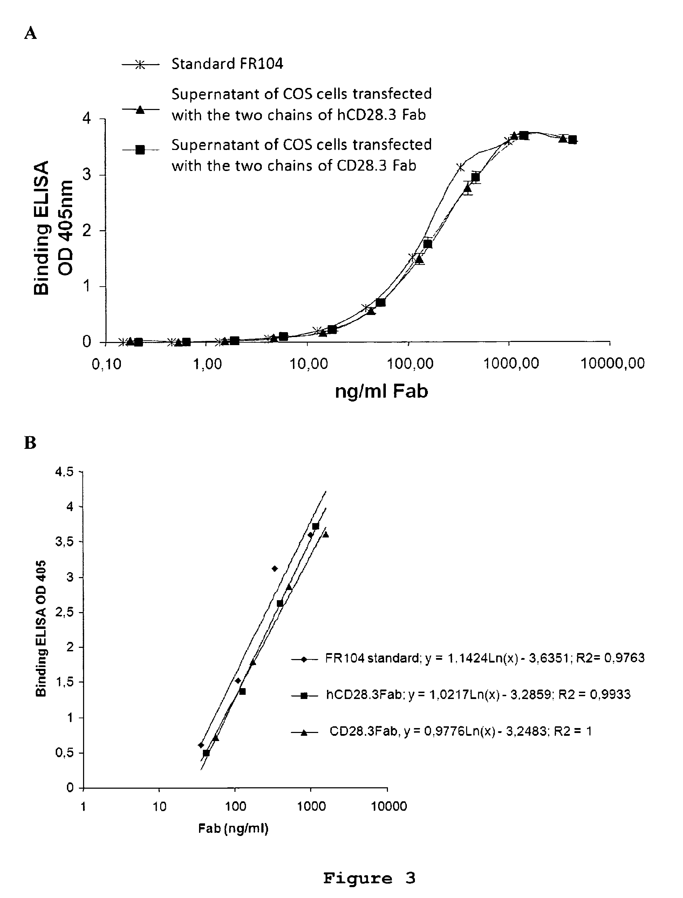

The results are shown on Figure 3 and Table I.

Figure 3A shows the optical density at 405 nm for increasing concentrations of FR104, hCD28.3 Fab or CD28.3 Fab in the Binding ELISA.

Figure 3B shows the calculation of the regression curves, allowing for determining comparative AC50 values.

Table I below summarises the OD50, the equation, and the ED50 for the standard FR104, and the Fab fragments VH-wild type + VL-wild type and hCD28.3

**Table I**

| | OD50 | Equation | AC50 |
|---|---|---|---|
| Std FR104 | 1,792 | y = 1,1424Ln(x) - 3,6351 | 115 |
| CD28.3 Fab | 1,82 | y = 0,9776Ln(x) - 3,2483 | 162 |
| hCD28.3 Fab | 1,804 | y = 1,0217Ln(x) - 3,2859 | 151 |

These results show that 50% of the binding activity to CD28 could be reached at a concentration similar for Fab fragments VH- wild type + VL- wild type (CD28.3 Fab) and hCD28.3 Fab. The concentration is slightly lower for the standard, probably because it is purified before the assay. Thus hCD28.3 retains the CD28-binding properties of the wild type VH and VL sequences of CD28.

## Claims

1. An anti-CD28 humanized antibody, **characterised in that**:
- its heavy chain comprises a variable domain defined by the following polypeptide sequence: 
VQLQQSGAELKKPGASVKVSCKASGYTFTEYIIHWIKLRSGQGLEWI
GWFYPGSNDIQYNAKFKGKATLTADKSSSTVYMELTGLTPEDSAVYFCARRDDFSG
YDALPYWGQGTLVTVSA (SEQ ID NO: 1);
- its light chain comprises a variable domain defined by the following polypeptide sequence: 
DIQMTQSPSSLSASVGDRVTITCKTNENIYSNLAWYQQKDGKSPQLL
IYAATHLVEGVPSRFSGSGSGTQYSLTISSLQPEDFGNYYCQHFWGTPXTFGGGTKLEI
 KR (SEQ ID NO: 2), wherein X = C or A.

2. An antibody of claim 1, which is a monovalent antibody.

3. A polynucleotide selected among:
a) a polynucleotide encoding a polypeptide of SEQ ID NO: 1;
b) a polynucleotide encoding a polypeptide of SEQ ID NO: 2;
c) a polypeptide encoding an antibody of any of claims 1 or 2.

4. A therapeutic composition comprising an antibody of any of claims 1 or 2.

5. An antibody of any of claims 1 or 2, for treating a pathological condition selected from the group consisting of transplant rejection, graft-versus-host disease, T-lymphocyte-mediated autoimmune diseases, allergic phenomena and chronic inflammatory diseases.
